# EUROPEAN PATENT APPLICATION

(11) **EP 2 995 670 A1**
(43) Date of publication of application: **16.03.2016**
(21) Application number: 15184587.2
(22) Date of filing: 10.09.2015
(51) Int. Cl.: C09K 8/584, A01N 41/04, A61K 8/46, C07C 309/04

(54) **ALKANE SULFONIC ACID OR SULFONATE COMPOSITION AND USE THEREOF IN ENHANCED OIL RECOVERY**

(30) Priority: 12.09.2014 EP 14184568
(71) Applicant: Shell Internationale Research Maatschappij B.V., 2596 HR Den Haag (NL)
(72) Inventor: MUL, Pim, 1031HW AMSTERDAM (NL); ROMMENS, Hidde Cornelis Willem, 1031 HW AMSTERDAM (NL)
(74) Representative: Matthezing, Robert Maarten

(57) **Abstract**

The invention relates to an alkane sulfonic acid or alkane sulfonate composition, which composition comprises an alkane substituted by a sulfonic acid or sulfonate group, wherein the alkane has an average carbon number in the range of from 16 to 30. Further, the invention relates to a method of treating a hydrocarbon containing formation using such composition as surfactant.

## Description

### Field of the invention

The present invention relates to an alkane sulfonic acid or sulfonate composition, and to a method of treating a hydrocarbon containing formation using an alkane sulfonic acid or sulfonate composition as surfactant.

### Background of the invention

It is known to make alkane sulfonic acids from alkanes (paraffins) by means of sulfoxidation of the alkanes, mostly linear alkanes (n-paraffins) which are usually in the range of C₁₂ to C₁₈. In such sulfoxidation, the alkane is reacted with sulfur dioxide (SO₂) and oxygen (O₂) in the presence of water whilst irradiating with ultraviolet (UV) light. By such irradiation, triplet sulfur dioxide is formed which in turn initiates the formation of alkyl radicals which are mostly secondary alkyl radicals. The alkyl radicals react with sulfur dioxide and then with oxygen, thereby forming radicals of the formula RS(=O)₂OO·. Abstraction of a hydrogen atom by such radical results in the formation of an alkyl radical and RS(=O)₂OOH. The latter peroxysulfonic acid is reduced by sulfur dioxide in the presence of water into the corresponding alkane sulfonic acid (RSO₃H) and sulfuric acid (H₂SO₄). The overall reaction is:

RH + 2SO₂ + O₂ + H₂O → RSO₃H + H₂SO₄

The alkane sulfonic acid (RSO₃H) may be converted into an anionic alkane sulfonate by reaction with a base, such as for example sodium hydroxide:

RSO₃H + NaOH → RSO₃Na + H₂O

Further, it is known to make alkane sulfonyl chlorides from alkanes (paraffins) by means of sulfochlorination of the alkanes and to hydrolyse such alkane sulfonyl chlorides into the corresponding alkane sulfonic acids. In such sulfochlorination, the alkane is reacted with sulfur dioxide (SO₂) and chlorine (Cl₂) whilst irradiating with visible light. By such irradiation, chlorine is dissociated into two chlorine radicals. Abstraction of hydrogen atoms by the chlorine radicals results in the formation of alkyl radicals which are mostly secondary alkyl radicals. Such alkyl radical reacts with sulfur dioxide and then with chlorine, thereby forming an alkane sulfonyl chloride (RSO₂Cl) and a chlorine radical. The overall reaction is:

RH + SO₂ + Cl₂ → RSO₂Cl + HCl

Using water, the alkane sulfonyl chloride (RSO₂Cl) may be hydrolysed into the corresponding alkane sulfonic acid (RSO₃H):

RSO₂Cl + H₂O → RSO₃H + HCl

Alternatively, the alkane sulfonyl chloride (RSO₂Cl) may be saponified into the corresponding anionic alkane sulfonate, using for example sodium hydroxide:

RSO₂Cl + 2NaOH → RSO₃Na + NaCl + H₂O

Such processes for preparing alkane sulfonic acids and sulfonates from alkanes involving sulfoxidation or sulfochlorination of the alkanes are for example described by H.G. Hauthal in "Alkanesulfonates" (Chapter 4, pages 143-221) in "Anionic surfactants: organic chemistry", edited by H.W. Stache (Surfactant science series, volume 56), Marcel Dekker, Inc., New York, 1995.

Commercial products comprising secondary alkane sulfonates (SAS) obtained by sulfoxidation of n-paraffins and subsequent neutralisation (reaction with a base) are for example the Hostapur® SAS products produced by Clariant (taken over by Weylchem per 1 January 2014). Some commercially available grades are Hostapur SAS 30, Hostapur SAS 60, Hostapur SAS 93 and Hostapur SAS 93 G, wherein said numbers 30, 60 and 93 refer to the active matter content (in %). These products are described in a brochure entitled "Hostapur ® SAS - A traditional specialty for innovative cleaner" by Clariant International Ltd., Muttenz, Switzerland, pages 1-56, 2013.

As appears from said Clariant brochure, the above-mentioned Hostapur SAS products are so-called sodium C14-17 secondary alkane sulfonates. Further, these products have been made by sulfoxidation of a mixture of n-paraffins showing the following carbon number distribution: <C₁₃: max. 1%; C₁₃-C₁₅ : about 58%; C₁₆-C₁₇: about 39%; and >C₁₇: max. 2%. The Hostapur SAS 60 product is also described in the above-mentioned Hauthal reference under "Hoechst Light-Water Technology" (pages 147-149), wherein it is mentioned that it would have a "mean carbon chain length" of 15.

Still further, it is known to use alkane sulfonates as surfactants in methods of treating hydrocarbon containing formations, such as oil containing formations, in order to recover hydrocarbons therefrom. In the above-mentioned Clariant brochure, it is suggested that Hostapur SAS is used in the "tertiary recovery of oil". Further, the following publications disclose the use of alkane sulfonates as surfactants in such methods: EP0353469A1, EP0302404A1, US4842067, US4722396 and US4295980.

Hydrocarbons, such as oil, may be recovered from hydrocarbon containing formations (or reservoirs) by penetrating the formation with one or more wells, which may allow the hydrocarbons to flow to the surface. A hydrocarbon containing formation may have one or more natural components that may aid in mobilising hydrocarbons to the surface of the wells. For example, gas may be present in the formation at sufficient levels to exert pressure on the hydrocarbons to mobilise them to the surface of the production wells. These are examples of so-called "primary oil recovery".

However, reservoir conditions (for example permeability, hydrocarbon concentration, porosity, temperature, pressure, composition of the rock) can significantly impact the economic viability of hydrocarbon production from any particular hydrocarbon containing formation. Furthermore, the above-mentioned natural pressure-providing components may become depleted over time, often long before the majority of hydrocarbons have been extracted from the reservoir. Therefore, supplemental recovery processes may be required and used to continue the recovery of hydrocarbons, such as oil, from the hydrocarbon containing formation. Such supplemental oil recovery is often called "secondary oil recovery" or "tertiary oil recovery". Examples of known supplemental processes include waterflooding, polymer flooding, gas flooding, alkali flooding, thermal processes, solution flooding, solvent flooding, or combinations thereof.

In recent years there has been increased activity in developing new and improved methods of chemical Enhanced Oil Recovery (cEOR) for maximising the yield of hydrocarbons from a subterranean reservoir. In surfactant cEOR, the mobilisation of residual oil is achieved through surfactants which generate a sufficiently low crude oil / water interfacial tension (IFT) to give a capillary number large enough to overcome capillary forces and allow the oil to flow (Lake, Larry W., "Enhanced oil recovery", PRENTICE HALL, Upper Saddle River, New Jersey, 1989, ISBN 0-13-281601-6).

However, different reservoirs can have different characteristics (for example composition of the rock, crude oil type, temperature, water composition - salinity, hardness etc.), and therefore, it is desirable that the structures and properties of the added surfactant(s) be matched to the particular conditions of a reservoir to achieve the required low IFT. In addition, other important criteria may have to be fulfilled, such as low rock retention or adsorption, compatibility with polymer, thermal and hydrolytic stability and acceptable cost (including ease of commercial scale manufacture).

In the present invention, it is desired to provide compositions and methods for cEOR utilising alkane sulfonic acids or sulfonates as surfactant. More in particular, to provide alkane sulfonic acids or sulfonates which have an improved cEOR performance, for example in terms of reducing the interfacial tension (IFT), as already described above. Further cEOR performance parameters of potential relevance are optimal salinity and aqueous solubility at such optimal salinity. By "optimal salinity", reference is made to the salinity of the brine present in a mixture comprising said brine (a salt-containing aqueous solution), the hydrocarbons (e.g. oil) and the surfactant(s), at which salinity said IFT is lowest. The salinity of the brine as present in oil-containing reservoirs may vary broadly, but in many reservoirs the brine salinity is between 1 and 5 wt.% of NaCl or between 1 and 10 wt.% of NaCl. Therefore, it is generally preferred that the optimal salinity of a surfactant composition likewise is between 1 and 10 wt.% of NaCl. In addition, it is preferred that at or close to such optimal salinity, the aqueous solubility of the surfactant is sufficient to good. Thus, in the present invention, it is desired to improve one or more of these cEOR performance parameters for alkane sulfonic acids or sulfonates.

### Summary of the invention

Surprisingly, it was found that an alkane sulfonic acid or alkane sulfonate composition having such improved cEOR performance parameter(s) is a composition which comprises an alkane substituted by a sulfonic acid or sulfonate group, wherein the alkane has an average carbon number in the range of from 16 to 30.

Accordingly, the present invention relates to an alkane sulfonic acid or alkane sulfonate composition as described above.

Further, the present invention relates to a method of treating a hydrocarbon containing formation, comprising the following steps:
a) providing the composition as described above to at least a portion of the hydrocarbon containing formation; and
b) allowing the alkane sulfonic acid or alkane sulfonate from the composition to interact with the hydrocarbons in the hydrocarbon containing formation.

### Brief description of the drawings

Figure 1 relates to an embodiment for application in cEOR.
Figure 2 relates to another embodiment for application in cEOR.

### Detailed description of the invention

In one aspect, the present invention relates to an alkane sulfonic acid or alkane sulfonate composition, which composition comprises an alkane substituted by a sulfonic acid or sulfonate group, wherein the alkane has an average carbon number in the range of from 16 to 30.

Thus, the composition of the present invention is an alkane sulfonic acid or alkane sulfonate composition, which comprises an alkane sulfonic acid or an alkane sulfonate. An alkane sulfonic acid is an alkane substituted by one or more sulfonic acid groups. An alkane sulfonate is an alkane substituted by one or more sulfonate groups.

It has appeared that the relatively high carbon number for the alkane, that is substituted by a sulfonic acid or sulfonate group, in the composition of the present invention results in advantageous performance properties, especially in a case where the composition of the present invention is to be used in enhanced oil recovery (cEOR). More in particular, it was surprisingly found that by using the composition of the present invention, wherein said alkane has an average carbon number in the range of from 16 to 30, improved cEOR performance parameters were obtained.

Thus, the composition of the present invention comprises an alkane sulfonic acid or alkane sulfonate. Said alkane sulfonic acid is prepared from an alkane and said alkane sulfonate is prepared from said alkane sulfonic acid. Within the present specification, an alkane and an alkane sulfonic acid or sulfonate comprise a mixture of alkane molecules and a mixture of alkane sulfonic acid or sulfonate molecules, respectively. That is to say, within the present specification, "alkane" as such refers to a mixture of alkane molecules whereas "alkane molecule" refers to one of the components from such mixture. Analogously, within the present specification, "alkane sulfonic acid" or "alkane sulfonate" as such refers to a mixture of alkane sulfonic acid or sulfonate molecules whereas "alkane sulfonic acid molecule" or "alkane sulfonate molecule" refers to one of the components from such mixture. Said molecules differ from each other for example in terms of carbon number and/or branching degree.

Branched alkane sulfonic acid or sulfonate molecules are alkane sulfonic acid or sulfonate molecules derived from alkane molecules which comprise one or more branches. Linear alkane sulfonic acid or sulfonate molecules are alkane sulfonic acid or sulfonate molecules derived from alkane molecules which are linear, that is to say which comprise no branches (unbranched alkane molecules). An alkane may be a mixture of linear alkane molecules and branched alkane molecules. Analogously, an alkane sulfonic acid or sulfonate may be a mixture of linear alkane sulfonic acid or sulfonate molecules and branched alkane sulfonic acid or sulfonate molecules.

An alkane and alkane sulfonic acid or sulfonate may be characterised by their carbon number and/or linearity. In case reference is made to an average carbon number, this means that the alkane or alkane sulfonic acid or sulfonate in question is a mixture of molecules which differ from each other in terms of carbon number.

Within the present specification, said average carbon number is determined by multiplying the number of carbon atoms of each molecule by the weight fraction of that molecule and then adding the products, resulting in a weight average carbon number. The average carbon number may be determined by gas chromatography (GC) analysis of the alkane.

Within the present specification, linearity is determined by dividing the weight of linear molecules by the total weight of branched, linear and cyclic molecules. Substituents (like the sulfonate group in alkane sulfonates) on the carbon chain are not seen as branches. The linearity may be determined by gas chromatography (GC) analysis of the alkane.

In the present invention, the alkane sulfonic acid or alkane sulfonate composition comprises an alkane substituted by a sulfonic acid or sulfonate group. Preferably, said alkane is linear. Preferably at least 60 wt.%, more preferably at least 70 wt.%, more preferably at least 80 wt.%, most preferably at least 90 wt.% of the alkane is linear. For example, 60 to 100 wt.%, more suitably 70 to 99 wt.%, most suitably 80 to 99 wt.% of the alkane may be linear.

Further, preferably, said alkane is not substituted by groups other than sulfonic acid and sulfonate groups. Further, preferably, said alkane does not contain unsaturated bonds. Further, in the present invention, said alkane has an average carbon number in the range of from 16 to 30. Further, preferably, said average carbon number may be in the range of from 16 to 28, or 16 to 25, or 17 to 25, or 18 to 25, or 18 to 24, or 18 to 23, or 18 to 22, or 19 to 23, or 20 to 22. Further, in the present invention, said average carbon number is at least 16, preferably at least 17, more preferably at least 18, more preferably at least 19, most preferably at least 20. Still further, in the present invention, said average carbon number is at most 30, preferably at most 28, more preferably at most 26, more preferably at most 25, more preferably at most 24, more preferably at most 23, most preferably at most 22.

Still further, preferably, said alkane may have a carbon number distribution within broad ranges. For example, in the present invention, one or more alkane sulfonic acids or alkane sulfonates may be selected from the group consisting of C₁₈₋₂₀ AS, C₁₈₋₂₃ AS and C₁₉₋₂₄ AS, wherein "AS" stands for "alkane sulfonic acid" or "alkane sulfonate". More preferably, one or more alkane sulfonic acids or alkane sulfonates are selected from the group consisting of C₁₈₋₂₀ AS and C₁₈₋₂₃ AS, most preferably C₁₈₋₂₃ AS.

"C₁₄₋₁₇ alkane sulfonic acid" or "C₁₄₋₁₇ alkane sulfonate" (C₁₄₋₁₇ AS) as used herein means a mixture of alkane sulfonic acids or alkane sulfonates, respectively, wherein the mixture has an average carbon number of from 14.5 to 16.5 and at least 50% by weight, preferably at least 70% by weight, more preferably at least 80% by weight, most preferably at least 90% by weight, of the alkane sulfonic acids or alkane sulfonates in the mixture contain from 14 to 17 carbon atoms.

"C₁₈₋₂₀ alkane sulfonic acid" or "C₁₈₋₂₀ alkane sulfonate" (C₁₈₋₂₀ AS) as used herein means a mixture of alkane sulfonic acids or alkane sulfonates, respectively, wherein the mixture has an average carbon number of from 18.2 to 19.2 and at least 50% by weight, preferably at least 70% by weight, more preferably at least 80% by weight, most preferably at least 90% by weight, of the alkane sulfonic acids or alkane sulfonates in the mixture contain from 18 to 20 carbon atoms.

Preferably 0 to 6, more preferably 0 to 4, most preferably 0 to 2 wt.% of said C₁₈₋₂₀ AS contains 16 or less carbon atoms. Preferably 0 to 8, more preferably 0 to 6, most preferably 0 to 5 wt.% of said C₁₈₋₂₀ AS contains 17 carbon atoms. Preferably 25 to 65, more preferably 35 to 55, most preferably 40 to 50 wt.% of said C₁₈₋₂₀ AS contains 18 carbon atoms. Preferably 20 to 50, more preferably 25 to 45, most preferably 30 to 40 wt.% of said C₁₈₋₂₀ AS contains 19 carbon atoms. Preferably 6 to 30, more preferably 10 to 26, most preferably 13 to 23 wt.% of said C₁₈₋₂₀ AS contains 20 carbon atoms. Preferably 0 to 10, more preferably 0 to 8, most preferably 0 to 7 wt.% of said C₁₈₋₂₀ AS contains 21 carbon atoms. Preferably 0 to 6, more preferably 0 to 4, most preferably 0 to 2 wt.% of said C₁₈₋₂₀ AS contains 22 or more carbon atoms.

"C₁₈₋₂₃ alkane sulfonic acid" or "C₁₈₋₂₃ alkane sulfonate" (C₁₈₋₂₃ AS) as used herein means a mixture of alkane sulfonic acids or alkane sulfonates, respectively, wherein the mixture has an average carbon number of from 19 to 21 and at least 50% by weight, preferably at least 70% by weight, more preferably at least 80% by weight, most preferably at least 90% by weight, of the alkane sulfonic acids or alkane sulfonates in the mixture contain from 18 to 23 carbon atoms.

Preferably 0 to 6, more preferably 0 to 4, most preferably 0 to 2 wt.% of said C₁₈₋₂₃ AS contains 16 or less carbon atoms. Preferably 0 to 8, more preferably 0 to 6, most preferably 0 to 5 wt.% of said C₁₈₋₂₃ AS contains 17 carbon atoms. Preferably 1 to 18, more preferably 4 to 15, most preferably 6 to 13 wt.% of said C₁₈₋₂₃ AS contains 18 carbon atoms. Preferably 4 to 28, more preferably 8 to 24, most preferably 11 to 21 wt.% of said C₁₈₋₂₃ AS contains 19 carbon atoms. Preferably 8 to 32, more preferably 12 to 28, most preferably 15 to 25 wt.% of said C₁₈₋₂₃ AS contains 20 carbon atoms. Preferably 10 to 40, more preferably 15 to 35, most preferably 20 to 30 wt.% of said C₁₈₋₂₃ AS contains 21 carbon atoms. Preferably 8 to 32, more preferably 12 to 28, most preferably 15 to 25 wt.% of said C₁₈₋₂₃ AS contains 22 carbon atoms. Preferably 0 to 15, more preferably 1 to 12, most preferably 2 to 10 wt.% of said C₁₈₋₂₃ AS contains 23 carbon atoms. Preferably 0 to 6, more preferably 0 to 4, most preferably 0 to 2 wt.% of said C₁₈₋₂₃ AS contains 24 or more carbon atoms.

"C₁₉₋₂₄ alkane sulfonic acid" or "C₁₉₋₂₄ alkane sulfonate" (C₁₉₋₂₄ AS) as used herein means a mixture of alkane sulfonic acids or alkane sulfonates, respectively, wherein the mixture has an average carbon number of from 20 to 22 and at least 50% by weight, preferably at least 70% by weight, more preferably at least 80% by weight, most preferably at least 90% by weight, of the alkane sulfonic acids or alkane sulfonates in the mixture contain from 19 to 24 carbon atoms.

Within the present specification, where reference is made to relative (e.g. weight) amounts of components in a mixture of components (e.g. said C₁₈₋₂₀ AS and C₁₈₋₂₃ AS mixtures of components having different carbon numbers), such relative amounts are to be selected such that the total amount of said mixture does not exceed 100%.

Further, for the alkanes which are substituted by one or more sulfonate groups (the alkane sulfonates), the cation may be any cation, such as an ammonium, alkali metal or alkaline earth metal cation, preferably an ammonium or alkali metal cation.

Generally, in alkane sulfonic acids and alkane sulfonates, the sulfonic acid or sulfonate group may be attached to a primary or secondary carbon atom. Preferably, in the present invention, the alkanes are substituted by sulfonic acid or sulfonate groups at a secondary carbon atom. However, in the present invention, the alkane sulfonic acid or sulfonate composition may comprise primary and secondary alkane sulfonic acids and sulfonates. Preferably, in the present invention, at most 5%, more preferably at most 2%, more preferably at most 1%, most preferably at most 0.5%, of the sulfonic acid and sulfonate groups is attached to a primary carbon atom.

In the present invention, the alkane sulfonic acid or sulfonate composition may comprise compounds of formulas (I) and (II) as a monosubstituted alkane and disubstituted alkane, respectively: 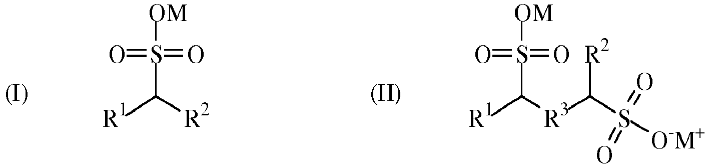 wherein the compound of formula (I) contains one sulfonic acid or sulfonate group and the compound of formula (II) contains two sulfonic acid or sulfonate groups; R¹ and R² are alkyl groups and R³ is a single bond or an alkylene group, which alkyl and alkylene groups are preferably linear, are preferably unsubstituted and preferably do not contain unsaturated bonds; and M is hydrogen or a cation as described above. Thus, the total carbon number for R¹ and R² and optionally R³ should be comprised within the above-mentioned average carbon number range of from 16 to 30 and may be comprised within the other above-mentioned, preferred narrower average carbon number ranges.

The alkane sulfonic acid or sulfonate composition of the present invention may comprise one or more other surfactants that are suitable for use in methods of chemical Enhanced Oil Recovery (cEOR), such as for example internal olefin sulfonates (IOS), alkoxylated alcohol sulfates, carboxylates and glycerol sulfonates, linear alkyl benzene sulfonates (LABS), and heavy alkyl benzene sulfonates (HABS). Furthermore, alkane sulfonic acids or sulfonates, wherein the alkanes do not have an average carbon number in the range of from 16 to 30, may also be added to the alkane sulfonic acid or sulfonate composition of the present invention.

In the present invention, a cosolvent (or solubilizer) may be added to increase the solubility of the alkane sulfonic acid or sulfonate surfactant in the alkane sulfonic acid or sulfonate composition of the present invention and/or in the below-mentioned injectable fluid comprising said composition. Suitable examples of cosolvents are polar cosolvents, including lower alcohols (for example sec-butanol and isopropyl alcohol) and polyethylene glycol. Any amount of cosolvent needed to dissolve all of the surfactant at a certain salt concentration (salinity) may be easily determined by a skilled person through routine tests.

Still further, the alkane sulfonic acid or sulfonate composition of the present invention may comprise a base (herein also referred to as "alkali"), preferably an aqueous soluble base, including alkali metal containing bases such as for example sodium carbonate and sodium hydroxide.

Alkane sulfonic acids and alkane sulfonates may be prepared from alkanes by any known method for preparing alkane sulfonic acids and alkane sulfonates, including methods which comprise alkane sulfoxidation or sulfochlorination, as discussed hereinabove. A suitable preparation method is for example disclosed in EP0599385A1 (Enichem), the disclosure of which is incorporated herein by reference. The starting alkanes may be obtained in any way. A suitable source of such alkanes (paraffins) is the Fischer-Tropsch process wherein a mixture of carbon monoxide and hydrogen is converted into liquid hydrocarbons (paraffins).

In another aspect, the present invention relates to a method of treating a hydrocarbon containing formation, comprising the following steps:
a) providing the composition as described above, that is to say an alkane sulfonic acid or alkane sulfonate composition, which composition comprises an alkane substituted by a sulfonic acid or sulfonate group, wherein the alkane has an average carbon number in the range of from 16 to 30, to at least a portion of the hydrocarbon containing formation; and
b) allowing the alkane sulfonic acid or alkane sulfonate from the composition to interact with the hydrocarbons in the hydrocarbon containing formation.

In the above-mentioned method of treating a hydrocarbon containing formation, the alkane sulfonic acid or alkane sulfonate (hereinafter also "AS") is applied in cEOR (chemical Enhanced Oil Recovery) at the location of the hydrocarbon containing formation, more in particular by providing the above-described AS composition to at least a portion of the hydrocarbon containing formation and then allowing the AS from said composition to interact with the hydrocarbons in the hydrocarbon containing formation. Said hydrocarbon containing formation may be a crude oil-bearing formation.

Normally, surfactants for enhanced hydrocarbon recovery are transported to a hydrocarbon recovery location and stored at that location in the form of an aqueous solution containing for example 30 to 35 wt.% of the surfactant. At the hydrocarbon recovery location, such solution would then be further diluted to a 0.05-2 wt.% solution, before it is injected into a hydrocarbon containing formation. By such dilution, an aqueous fluid is formed which fluid can be injected into the hydrocarbon containing formation, that is to say an injectable fluid. The water used in such dilution may originate from the formation from which hydrocarbons are to be recovered, which formation water may have to be pretreated, for example by removing divalent ions. Preferably, said water is brine, which is a monovalent salt (for example NaCl) containing aqueous solution.

The amount of AS surfactant, or the total amount of surfactants in case one or more other surfactants are used, in said injectable fluid, may be of from 0.05 to 2 wt.%, preferably 0.1 to 1.5 wt.%, more preferably 0.1 to 1.0 wt.%, most preferably 0.2 to 0.5 wt.%.

In the present invention, the temperature within the hydrocarbon containing formation may be between 10°C and 150°C, optionally between 30°C and 90°C. Further, in the present invention, the salinity of the water originating from the hydrocarbon containing formation may be between 0.5% and 20% or between 0.5% and 10% or between 1% and 6%.

Hydrocarbons may be produced from hydrocarbon containing formations through wells penetrating such formations. "Hydrocarbons" are generally defined as molecules formed primarily of carbon and hydrogen atoms such as oil and natural gas. Hydrocarbons may also include other elements, such as halogens, metallic elements, nitrogen, oxygen and/or sulfur. Hydrocarbons derived from a hydrocarbon containing formation may include kerogen, bitumen, pyrobitumen, asphaltenes, oils or combinations thereof. Hydrocarbons may be located within or adjacent to mineral matrices within the earth. Matrices may include sedimentary rock, sands, silicilytes, carbonates, diatomites and other porous media.

A "hydrocarbon containing formation" may include one or more hydrocarbon containing layers, one or more non-hydrocarbon containing layers, an overburden and/or an underburden. An overburden and/or an underburden includes one or more different types of impermeable materials. For example, overburden/underburden may include rock, shale, mudstone, or wet/tight carbonate (that is to say an impermeable carbonate without hydrocarbons). For example, an underburden may contain shale or mudstone. In some cases, the overburden/underburden may be somewhat permeable. For example, an underburden may be composed of a permeable mineral such as sandstone or limestone.

Properties of a hydrocarbon containing formation may affect how hydrocarbons flow through an underburden/overburden to one or more production wells. Properties include porosity, permeability, pore size distribution, surface area, salinity or temperature of formation. Overburden/underburden properties in combination with hydrocarbon properties, capillary pressure (static) characteristics and relative permeability (flow) characteristics may affect mobilisation of hydrocarbons through the hydrocarbon containing formation.

Fluids (for example gas, water, hydrocarbons or combinations thereof) of different densities may exist in a hydrocarbon containing formation. A mixture of fluids in the hydrocarbon containing formation may form layers between an underburden and an overburden according to fluid density. Gas may form a top layer, hydrocarbons may form a middle layer and water may form a bottom layer in the hydrocarbon containing formation. The fluids may be present in the hydrocarbon containing formation in various amounts. Interactions between the fluids in the formation may create interfaces or boundaries between the fluids. Interfaces or boundaries between the fluids and the formation may be created through interactions between the fluids and the formation. Typically, gases do not form boundaries with other fluids in a hydrocarbon containing formation. A first boundary may form between a water layer and underburden. A second boundary may form between a water layer and a hydrocarbon layer. A third boundary may form between hydrocarbons of different densities in a hydrocarbon containing formation.

Production of fluids may perturb the interaction between fluids and between fluids and the overburden/underburden. As fluids are removed from the hydrocarbon containing formation, the different fluid layers may mix and form mixed fluid layers. The mixed fluids may have different interactions at the fluid boundaries. Depending on the interactions at the boundaries of the mixed fluids, production of hydrocarbons may become difficult.

Quantification of energy required for interactions (for example mixing) between fluids within a formation at an interface may be difficult to measure. Quantification of energy levels at an interface between fluids may be determined by generally known techniques (for example spinning drop tensiometer). Interaction energy requirements at an interface may be referred to as interfacial tension. "Interfacial tension" as used herein, refers to a surface free energy that exists between two or more fluids that exhibit a boundary. A high interfacial tension value (for example greater than 10 dynes/cm) may indicate the inability of one fluid to mix with a second fluid to form a fluid emulsion. As used herein, an "emulsion" refers to a dispersion of one immiscible fluid into a second fluid by addition of a compound that reduces the interfacial tension between the fluids to achieve stability. The inability of the fluids to mix may be due to high surface interaction energy between the two fluids. Low interfacial tension values (for example less than 1 dyne/cm) may indicate less surface interaction between the two immiscible fluids. Less surface interaction energy between two immiscible fluids may result in the mixing of the two fluids to form an emulsion. Fluids with low interfacial tension values may be mobilised to a well bore due to reduced capillary forces and subsequently produced from a hydrocarbon containing formation. Thus, in surfactant cEOR, the mobilisation of residual oil is achieved through surfactants which generate a sufficiently low crude oil / water interfacial tension (IFT) to give a capillary number large enough to overcome capillary forces and allow the oil to flow.

Mobilisation of residual hydrocarbons retained in a hydrocarbon containing formation may be difficult due to viscosity of the hydrocarbons and capillary effects of fluids in pores of the hydrocarbon containing formation. As used herein "capillary forces" refers to attractive forces between fluids and at least a portion of the hydrocarbon containing formation. Capillary forces may be overcome by increasing the pressures within a hydrocarbon containing formation. Capillary forces may also be overcome by reducing the interfacial tension between fluids in a hydrocarbon containing formation. The ability to reduce the capillary forces in a hydrocarbon containing formation may depend on a number of factors, including the temperature of the hydrocarbon containing formation, the salinity of water in the hydrocarbon containing formation, and the composition of the hydrocarbons in the hydrocarbon containing formation.

As production rates decrease, additional methods may be employed to make a hydrocarbon containing formation more economically viable. Methods may include adding sources of water (for example brine, steam), gases, polymers or any combinations thereof to the hydrocarbon containing formation to increase mobilisation of hydrocarbons.

In the present invention, the hydrocarbon containing formation is thus treated with the diluted or not-diluted AS surfactant containing solution, as described above. Interaction of said solution with the hydrocarbons may reduce the interfacial tension of the hydrocarbons with one or more fluids in the hydrocarbon containing formation. The interfacial tension between the hydrocarbons and an overburden/underburden of a hydrocarbon containing formation may be reduced. Reduction of the interfacial tension may allow at least a portion of the hydrocarbons to mobilise through the hydrocarbon containing formation.

The ability of the AS surfactant containing solution to reduce the interfacial tension of a mixture of hydrocarbons and fluids may be evaluated using known techniques. The interfacial tension value for a mixture of hydrocarbons and water may be determined using a spinning drop tensiometer. An amount of the AS surfactant containing solution may be added to the hydrocarbon/water mixture and the interfacial tension value for the resulting fluid may be determined.

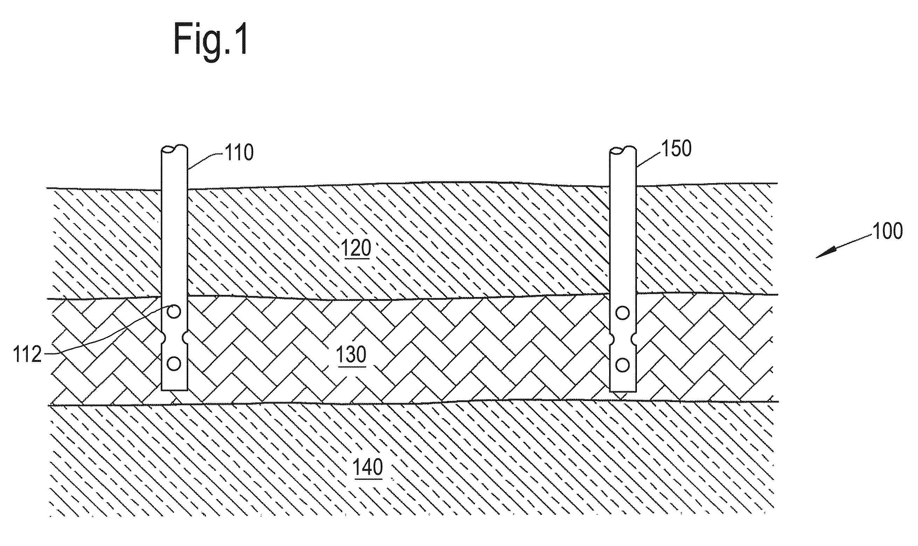

The AS surfactant containing solution, diluted or not diluted, may be provided (for example injected in the form of a diluted aqueous fluid) into hydrocarbon containing formation 100 through injection well 110 as depicted in Figure 1. Hydrocarbon containing formation 100 may include overburden 120, hydrocarbon layer 130 (the actual hydrocarbon containing formation), and underburden 140. Injection well 110 may include openings 112 (in a steel casing) that allow fluids to flow through hydrocarbon containing formation 100 at various depth levels. Low salinity water may be present in hydrocarbon containing formation 100.

The surfactant from the AS surfactant containing solution may interact with at least a portion of the hydrocarbons in hydrocarbon layer 130. This interaction may reduce at least a portion of the interfacial tension between one or more fluids (for example water, hydrocarbons) in the formation and the underburden 140, one or more fluids in the formation and the overburden 120 or combinations thereof.

The surfactant from the AS surfactant containing solution may interact with at least a portion of hydrocarbons and at least a portion of one or more other fluids in the formation to reduce at least a portion of the interfacial tension between the hydrocarbons and one or more fluids. Reduction of the interfacial tension may allow at least a portion of the hydrocarbons to form an emulsion with at least a portion of one or more fluids in the formation. The interfacial tension value between the hydrocarbons and one or more other fluids may be altered by the AS surfactant containing solution to a value of less than 0.1 dyne/cm or less than 0.05 dyne/cm or less than 0.001 dyne/cm.

At least a portion of the AS surfactant containing solution/hydrocarbon/fluids mixture may be mobilised to production well 150. Products obtained from the production well 150 may include components of the AS surfactant containing solution, methane, carbon dioxide, hydrogen sulfide, water, hydrocarbons, ammonia, asphaltenes or combinations thereof. Hydrocarbon production from hydrocarbon containing formation 100 may be increased by greater than 50% after the AS surfactant containing solution has been added to a hydrocarbon containing formation.

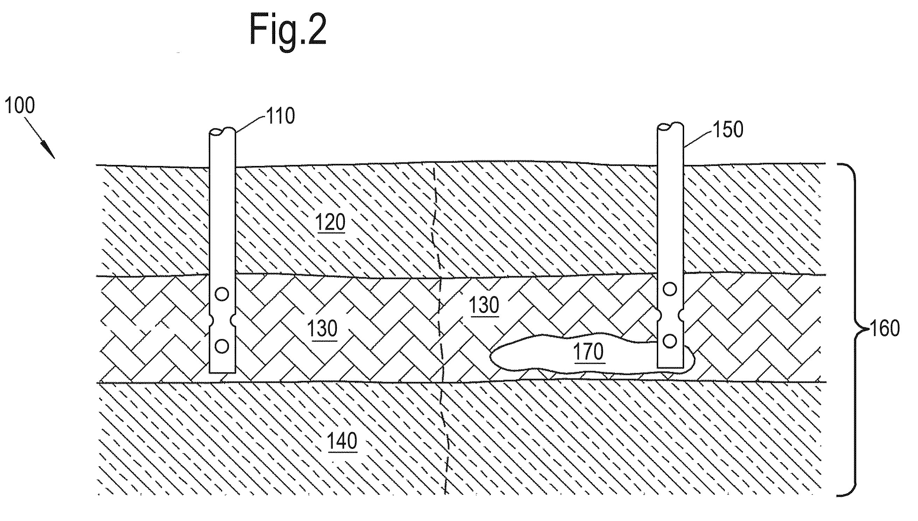

The AS surfactant containing solution, diluted or not diluted, may also be injected into hydrocarbon containing formation 100 through injection well 110 as depicted in Figure 2. Interaction of the surfactant from the AS surfactant containing solution with hydrocarbons in the formation may reduce at least a portion of the interfacial tension between the hydrocarbons and underburden 140. Reduction of at least a portion of the interfacial tension may mobilise at least a portion of hydrocarbons to a selected section 160 in hydrocarbon containing formation 100 to form hydrocarbon pool 170. At least a portion of the hydrocarbons may be produced from hydrocarbon pool 170 in the selected section of hydrocarbon containing formation 100.

It may be beneficial under certain circumstances that an aqueous fluid, wherein the AS surfactant containing solution is diluted, contains inorganic salt, such as sodium chloride, sodium hydroxide, potassium chloride, ammonium chloride, sodium sulfate or sodium carbonate. Such inorganic salt may be added separately from the AS surfactant containing solution or it may be included in the AS surfactant containing solution before it is diluted in water. The addition of the inorganic salt may help the fluid disperse throughout a hydrocarbon/water mixture and to reduce surfactant loss by adsorption onto rock. This enhanced dispersion may decrease the interactions between the hydrocarbon and water interface. The decreased interaction may lower the interfacial tension of the mixture and provide a fluid that is more mobile.

The invention is further illustrated by the following Examples.

### Examples

In the present Examples, some relevant cEOR performance parameters (cEOR = chemical Enhanced Oil Recovery) were measured for three different alkane sulfonate compositions.

The above-mentioned alkane sulfonate compositions were prepared by sulfoxidation of the alkane feedstock by a radical-initiated reaction with sulfur dioxide (SO₂) and oxygen (O₂) in the presence of water, thereby forming mainly secondary alkane sulfonic acids, which were then converted into the corresponding sulfonates by reaction with sodium hydroxide. Three types of alkane (paraffin) feedstocks were used, herein designated as "alkane I", "alkane II" and "alkane III", respectively. Properties of these feedstocks are shown in Table 1 below.

**Table 1**

| | Alkane I^{(*)} | Alkane II⁽³⁾ | Alkane III⁽³⁾ |
|---|---|---|---|
| Composition in terms of carbon number (wt.%) | | | |
| C13 | ≤C13: <1 | 0.00 | 0.00 |
| C14 | 39 | 0.00 | 0.00 |
| C15 | 32 | 0.00 | 0.01 |
| C16 | 19 | 0.01 | 0.12 |
| C17 | 9 | 2.20 | 2.61 |
| C18 | ≥C18: <1 | 43.56 | 9.13 |
| C19 | | 32.77 | 15.11 |
| C20 | | 17.18 | 21.77 |
| C21 | | 3.98 | 25.07 |
| C22 | | 0.21 | 19.54 |
| C23 | | 0.00 | 6.20 |
| C24 | | 0.00 | 0.40 |
| C25 | | 0.00 | 0.00 |
| Total | 100 | 99.91 | 99.96 |
| Weight average carbon number ⁽¹⁾ | 15.0 | 18.8 | 20.4 |
| Linearity(2) (wt.%) | - | 92.2 | 92.8 |
| Corresponding alkane sulfonate (AS) | C₁₄₋₁₇ AS; C₁₄₋₁₇ : 99 wt.% | C₁₈₋₂₀ AS; C₁₈₋₂₀: 93.51 wt.% | C₁₈₋₂₃ AS; C₁₈₋₂₃ : 96.82 wt.% |

| | | | |
|---|---|---|---|
| (1) "Weight average carbon number" = sum of the products obtained by multiplying the number of carbon atoms (above "carbon number") of each alkane molecule by the weight fraction of that molecule. (2) "Linearity" = (weight of linear molecules based on total weight of branched, linear and cyclic molecules)*100%. (3) Alkanes II and III are paraffins which were made by using the Fischer-Tropsch process wherein a mixture of carbon monoxide and hydrogen is converted into liquid hydrocarbons (paraffins). (*) = not according to the invention | | | |

The alkane sulfonates as prepared from alkanes I, II and III are herein designated as "C₁₄₋₁₇ AS", "C₁₈₋₂₀ AS" and "C₁₈₋₂₃ AS", respectively. Said alkane sulfonates were provided in the form of aqueous C₁₄₋₁₇ AS, C₁₈₋₂₀ AS and C₁₈₋₂₃ AS compositions, respectively, of which the alkane sulfonate (active matter) content was 60, 20 and 25 wt.%, respectively.

The C₁₈₋₂₀ AS and C₁₈₋₂₃ AS compositions were treated by extraction. In the extraction treatment, the following procedure was performed. 200 ml of a 50/50 (vol./vol.) mixture of two solvents, namely water and 2-butanol, were added to 10 g of the C₁₄₋₁₇ AS composition. The resulting solution was transferred into a separation funnel. A small amount of water was added to compensate for the better solubility of water in 2-butanol compared to the solubility of 2-butanol in water, to arrive at about equal volumes of two separate liquid phases upon extraction by thorough shaking of the separation funnel, one of which 2 phases was more polar than the other. The temperature and pressure during the extraction were ambient temperature and pressure. After the extraction, the less polar and more polar phases were separated from each other. The more polar, water-rich lower layer was removed and discarded. Finally, the volatile components (2-butanol and water) were removed from the remaining 2-butanol-rich phase using a rotating film evaporator. In total, the C₁₈₋₂₀ AS and C₁₈₋₂₃ AS compositions were treated by extraction 4 times. In the 2^{nd} and further extractions, 2-butanol and water were added to said remaining 2-butanol-rich phase, in such amounts resulting in 2 phases in a 50/50 (vol./vol.) ratio, after which the extraction was performed as mentioned hereinbefore.

In Table 2 below, some cEOR performance parameters are included for the above-mentioned alkane sulfonates. These parameters relate to a desired use of the alkane sulfonates as a surfactant in chemical Enhanced Oil Recovery (cEOR). More in particular, so-called phase behaviour tests were conducted, as described below, in order to determine the optimal salinity and the solubilisation parameter, the values for which are shown in Table 2.

In order to determine said optimal salinity and solubilisation parameter, aqueous solutions comprising the alkane sulfonate and having different salinities were prepared. In tubes, the aqueous solutions were mixed with n-octane in a volume ratio of 1:1 and the system was allowed to equilibrate for days or weeks at 90 °C. The alkane n-octane was used as a model oil for the screening tests, to represent a simple crude oil.

Microemulsion phase behaviour tests were carried out to screen the surfactants for their potential to mobilize residual oil by means of lowering the interfacial tension (IFT) between the oil and water. Microemulsion phase behaviour was first described by Winsor in "Solvent properties of amphiphilic compounds", Butterworths, London, 1954. The following categories of emulsions were distinguished by Winsor: "type I" (oil-in-water emulsion), "type II" (water-in-oil emulsion) and "type III" (emulsions comprising a bicontinuous oil/water phase). A Winsor Type III emulsion is also known as an emulsion which comprises a so-called "middle phase" microemulsion. A microemulsion is characterised by having the lowest IFT between the oil and water for a given oil/water mixture.

For anionic surfactants, increasing the salinity (salt concentration) of an aqueous solution comprising the surfactant(s) causes a transition from a Winsor type I emulsion to a type III and then to a type II. Optimal salinity is defined as the salinity where equal amounts of oil and water are solubilised in the middle phase (type III) microemulsion. The oil solubilisation ratio is the ratio of oil volume (Vₒ) to neat surfactant volume (Vₛ) and the water solubilisation ratio is the ratio of water volume (V_{w}) to neat surfactant volume (Vₛ). The intersection of Vₒ/Vₛ and V_{w}/Vₛ as salinity is varied, defines (a) the optimal salinity and (b) the solubilisation parameter at the optimal salinity. It has been established by Huh that IFT is inversely proportional to the square of the solubilisation parameter (Huh, "Interfacial tensions and solubilizing ability of a microemulsion phase that coexists with oil and brine", J. Colloid and Interface Sci., September 1979, p. 408-426). A high solubilisation parameter, and consequently a low IFT, is advantageous for mobilising residual oil via surfactant EOR. That is to say, the higher the solubilisation parameter the more "active" the surfactant.

The detailed microemulsion phase test method used in these Examples has been described previously, by Barnes et al. under Section 2.1 "Glass pressure tube test" in "Development of Surfactants for Chemical Flooding at Difficult Reservoir Conditions", SPE 113313, 2008, p. 1-18. In summary, this test provides two important data:
(a) the optimal salinity, expressed as wt.% NaCl;
(b) the solubilisation parameter (SP; in ml/ml; assumption: density surfactant = 1 g/ml) at the optimal salinity (this usually takes several days or weeks to allow the phases to settle at equilibrium), wherein the interfacial tension (IFT, in mN/m) is calculated from the solubilisation parameter using the "Huh" equation IFT=0.3/SP² as referred to above.

**Table 2**

| Alkane sulfonate | Optimal salinity⁽¹⁾ wt.% NaCl | SP ⁽¹⁾ ml/ml |
|---|---|---|
| C₁₄₋₁₇ AS (untreated) ⁽*⁾ | 10.0+/-0.5 | 5-7 |
| C₁₈₋₂₀ AS (untreated) | 5.0+/-0.5 | 12-18 |
| C₁₈₋₂₀ AS (extracted 4 times) | 1.1+/-0.2 | 35-45 |
| C₁₈₋₂₃ AS (extracted 4 times) | 0.9+/-0.2 | 60-80 |

| | | |
|---|---|---|
| (1) The aqueous solution used for determining the optimal salinity and the solubilisation parameter (SP) comprised 2 wt.% of the alkane sulfonate, except for C₁₈₋₂₃ AS in which case said concentration was 1 wt.%. (*) = not according to the invention | | |

It appears from Table 2 that alkane sulfonate compositions according to the invention, including the above-mentioned C₁₈₋₂₀ AS and C₁₈₋₂₃ AS compositions, wherein the alkane, that is substituted by one or more sulfonate groups, has an average carbon number in the range of from 16 to 30, namely 18.8 and 20.4 (see Table 1), respectively, can be advantageously used as a surfactant in chemical Enhanced Oil Recovery (cEOR) which requires them to lower the interfacial tension (IFT) between oil and water. Still further, for the C₁₈₋₂₀ AS and C₁₈₋₂₃ AS compositions according to the invention, even a stronger ability to lower the IFT is demonstrated by an increased solubilisation parameter (SP), as compared to the C₁₄₋₁₇ AS composition which is not according to the invention since the average carbon number was below 16, namely 15.0 (see Table 1).

Furthermore, it appears from Table 2 that, advantageously, the optimal salinity for all of the above-mentioned alkane sulfonate compositions according to the invention, having an average carbon number in the range of from 16 to 30, is decreased which makes them suitable to be used in cEOR methods wherein the crude oil containing reservoir or formation in question contains water (brine) which has a relatively low salinity, as compared to the C₁₄₋₁₇ AS composition which is not according to the invention.

In case the optimal salinity of an alkane sulfonate composition according to the invention would be too high as compared to the salinity of the water (brine) as contained in the crude oil containing reservoir or formation in question, said alkane sulfonates can still be advantageously used in combination with surfactants having a lower optimal salinity. Vice versa, in case the optimal salinity of an alkane sulfonate composition according to the invention would be too low, said alkane sulfonates can still be advantageously used in combination with surfactants having a higher optimal salinity.

## Claims

1. Alkane sulfonic acid or alkane sulfonate composition, which composition comprises an alkane substituted by a sulfonic acid or sulfonate group, wherein the alkane has an average carbon number in the range of from 16 to 30.

2. Alkane sulfonic acid or alkane sulfonate composition according to claim 1, wherein the alkane has an average carbon number in the range of from 16 to 25, more preferably 18 to 24, more preferably 18 to 23, most preferably 18 to 22.

3. Alkane sulfonic acid or alkane sulfonate composition according to claim 1 or 2, wherein one or more alkane sulfonic acids or alkane sulfonates are selected from the group consisting of C₁₈₋₂₀ AS and C₁₈₋₂₃ AS, preferably C₁₈₋₂₃ AS, wherein "AS" stands for "alkane sulfonic acid" or "alkane sulfonate".

4. Alkane sulfonic acid or alkane sulfonate composition according to any one of the preceding claims, which composition further comprises one or more surfactants selected from the group consisting of internal olefin sulfonates (IOS), alkoxylated alcohol sulfates, carboxylates and glycerol sulfonates, linear alkyl benzene sulfonates (LABS), and heavy alkyl benzene sulfonates (HABS).

5. A method of treating a hydrocarbon containing formation, comprising the following steps:
a) providing an alkane sulfonic acid or alkane sulfonate composition, which composition comprises an alkane substituted by a sulfonic acid or sulfonate group, wherein the alkane has an average carbon number in the range of from 16 to 30, to at least a portion of the hydrocarbon containing formation; and
b) allowing the alkane sulfonic acid or alkane sulfonate from the composition to interact with the hydrocarbons in the hydrocarbon containing formation.

6. Method according to claim 5, wherein the alkane has an average carbon number in the range of from 16 to 25, more preferably 18 to 24, more preferably 18 to 23, most preferably 18 to 22.

7. Method according to claim 5 or 6, wherein one or more alkane sulfonic acids or alkane sulfonates are selected from the group consisting of C₁₈₋₂₀ AS and C₁₈₋₂₃ AS, preferably C₁₈₋₂₃ AS, wherein "AS" stands for "alkane sulfonic acid" or "alkane sulfonate".

8. Method according to any one of claims 5-7, which composition further comprises one or more surfactants selected from the group consisting of internal olefin sulfonates (IOS), alkoxylated alcohol sulfates, carboxylates and glycerol sulfonates, linear alkyl benzene sulfonates (LABS), and heavy alkyl benzene sulfonates (HABS).
